(19) **Europäisches Patentamt**

**European Patent Office**

**Office européen des brevets**

(11) **EP 1 150 675 B1**

(12) **EUROPEAN PATENT SPECIFICATION**

(45) Date of publication and mention
of the grant of the patent:
**01.02.2006 Bulletin 2006/05**

(51) Int Cl.:
*A61K 31/404* (2006.01)   *A61K 31/4178* (2006.01)
*A61K 31/439* (2006.01)   *A61K 47/10* (2006.01)
*A61K 47/12* (2006.01)   *A61K 47/14* (2006.01)
*A61K 9/70* (2006.01)   *A61P 1/08* (2006.01)

(21) Application number: **00904103.9**

(22) Date of filing: **09.02.2000**

(86) International application number:
**PCT/KR2000/000096**

(87) International publication number:
**WO 2000/047208 (17.08.2000 Gazette 2000/33)**

(54) **A TRANSDERMAL COMPOSITION OF AN ANTIVOMITING AGENT AND A PREPARATION CONTAINING THE SAME**

TRANSDERMALE ZUSAMMENSTELLUNG EINES ANTIBRECHMITTELSUND EINE ZUBEREITUNG DIE DASSELBE ENTHÄLT

COMPOSITION TRANSDERMIQUE D'UN AGENT ANTIEMETIQUE ET PREPARATION CONTENANT CETTE COMPOSITION

(84) Designated Contracting States:
**CH DE ES FR GB IT LI**

(30) Priority: **09.02.1999 KR 9904500**

(43) Date of publication of application:
**07.11.2001 Bulletin 2001/45**

(73) Proprietor: **Samyang Corporation
Chongno-ku
Seoul 110-470 (KR)**

(72) Inventors:
• **SEO, Bo Youn
Daejeon 305-390 (KR)**
• **CHO, Joong Woong
305-3 48 Daejeon (KR)**
• **CHOI, Young Kweon
Yusung-ku,
Daejeon 305-390 (KR)**
• **HWANG, Jun Seok
Daejeon 302-223 (KR)**

(74) Representative: **Reinhard - Skuhra - Weise & Partner
Friedrichstrasse 31
80801 München (DE)**

(56) References cited:
**EP-A1- 0 682 942          WO-A1-98/30244
WO-A1-98/53815          US-A- 5 908 619**

**Description**

**FIELD OF THE INVENTION**

The present invention relates to a transdermal composition of an antivomiting agent and a preparation containing the same which is capable of delivering the antivomiting agent efficiently over a period of a day or more without skin irritation.

**BACKGROUND OF THE INVENTION**

Vomiting is induced by many causes which include pathologic factors, e.g., damaged brain; physiologic factors, e.g., pregnancy; and therapeutic factors, e.g., chemotherapy. The chemotherapy involving the use of an anticancer agent, e.g., cisplatin, causes serious vomiting.

Vomiting is controlled by the emetic center located in cerebral medulla. A vomiting reflex is induced by abnormal activity of the visceral afferent neuron located in the abdominal vagus nerve which receives signals from the chemoreceptor trigger zone(CTZ) located in the emetic center: Thus, the vomiting process is mediated by neurotransmitters such as serotonin(5-hydroxytryptamine sub type 3, 5-HT3), acetylcholine, dopamine and histamine.

Accordingly, neutotransmitter antagonists have been employed as antivomiting agents, and particularly, widely used are serotonin antagonists, e.g., tropisetron, ondansetron, granisetron and dolasetron. Tropisetron, administered parenterally and orally in an amount of 5 mg per a day, is effective in treating acute vomiting caused by cisplatin, and ondansetron is also administered parenterally or orally. Such serotonin antagonists cause no serious extrapyramidal side effects such as tardive dyskinesia, acute myodystona, akathisia and tremor.

However, the oral administration of an antivomiting agent is not adequate for treating serious vomiting. Intravenous and instillative injection of an antivomiting agent is painful and limited to a hospital practice. Therefore, there has existed a need to develop an improved method for administering antivomiting agents.

A transdermal drug delivery system in general is one of controlled release systems which make it possible to maintain an effective drug concentration in the blood with one application. The transdermal delivery of a drug provides several advantages: it is easy to handle and capable of maintaining an effective drug level in the blood over an extended period; eliminates the fluctuation of the drug concentration in the blood typically seen when a drug is orally administered; is suitable for a drug having a short half-life; avoids initial degradation of a drug in the liver; and is easy to remove after a prescribed time.

However, an effective formulation for transdermal administration of an antivomiting agent has not yet been developed.

WO 9830244 discloses hydroalcoholic compositions useful for the enhancement of the transdermal delivery of a pharmaceutical agent. The composition includes a lower alcohol and water, a pharmaceutical agent and an emulsifier system. It describes on page 8, lines 10-14 that the emulsifier system functions as a penetration enhancer. Some fatty alcohols, fatty alcohol derivates etc. are disclosed as examples for an emulsifier.

EP 0682942 discloses a transdermal composition comprising a physiologically active substance, a monoterpene, a non-ionic surface active agent, ethanol and water (see claim 1).

However, it is not disclosed therein to use a mixture of 1 to 5% by weight of N,N-diethyl-m-toluamide and 1 to 5% by weight of glycerol monolaureate as a skin penetration enhancer

**SUMMARY OF THE INVENTION**

Accordingly, it is an object of the present invention to provide a pharmaceutical composition for transdermally administering an antivomiting agent which is capable of delivering the antivomiting agent efficiently to the blood over an extended period without skin irritation.

Another object of the present invention is to provide a preparation containing the pharmaceutical composition.

In accordance with the present invention, there is provided a transdermal composition comprising (a) a matrix containing (i) 20 to 80 % by weight of a hydrophilic organic solvent, (ii) a mixture of 1 to 5% by weight of N,N-diethyl-m-toluamide and 1 to 5% by weight of glycerol monolaureate as a skin penetration enhancer and (iii) 15 to 80 % by weight of water; and (b) 1 to 15 % by weight, based on the weight of the matrix, of an antivomiting agent selected from the group consisting of tropisetron, ondansetron, granisetron and pharmaceutically acceptable salts thereof.

**BRIEF DESCRIPTION OF THE DRAWINGS**

The above objects and features of the present invention wiU become apparent from the following description of preferred embodiinents taken in conjunction with the accompanying drawings, in which:

Fig. 1 shows a schematic representation of the reservoir patch in accordance with one embodiment of the present invention;

Fig. 2 depicts a schematic representation of another reservoir patch in accordance with another embodiment of the present invention; and

Fig. 3 is a schematic representation of the monolithic matrix patch of the present invention.

## DETAILED DESCRIPTION OF THE INVENTION

A transdermal composition of the present invention generally comprises a matrix comprising a hydrophilic organic solvent, a skin penetration enhancer, and water; and an antivomiting agent.

The antivomiting agent which is used in the present invention is tropisetron, ondansetron, granisetron or a mixture thereof. These antivomiting agents may be in the form of a free base or a pharmaceutically acceptable salt thereof. The antivomiting agent may be used in an amount ranging from 1 to 15 % by weight, preferably from 3 to 10 % by weight, based on the weight of the matrix.

To deliver an effective amount of an antivomiting agent transdermally, a composition for the transdermal administration has to provide a high skin penetration rate of the antivomiting drug. Formulas (I) to (V), which have been established based on *in vitro* thansdermal delivery data, and various pharmacokinetic parameters (*Drugs, 46,* 925-943 (1993)) are used to define an antivomiting transdermal system, e.g., dose, skin penetration rates and application area;

$$D_t = D_o[1-E] \qquad (I)$$

$$D_{SS} = J_S \cdot A \cdot t \qquad (II)$$

$$K_0 = Cl_T \cdot C_{SS} \qquad (III)$$

$$K_0 = J_S \cdot A \qquad (IV)$$

$$C_{SS} = J_S \cdot A / Cl_T \qquad (V)$$

wherein Dt represents the transdermal administration dose of a drug; $D_o$, oral administration dose; E is the drug extraction ratio; Dss, the amount of the drug penetrating the skin per unit time; A, the skin area; t, the time for the drug penetrating the skin; Js, the skin penetration rate of the drug at a steady state; $K_0$, the infusion rate of the drug; $C1_T$, total body clearance; and Css, the drug level in the blood at a steady state.

According to the above formula and various pharmacokinetic parameters, transdermal penetration rates of 200 to 600 $\mu$g/h and 600 to 800 $\mu$g/h must be established for tropisetron and ondansetron, respectively, when a plasma tropisetron level of 3 to 10 ng/ ml and a plasma ondansetron level of 10 to 30 ng/ml are to be achieved.

The skin penetration rates of higher than 20 $\mu$g/cm$^2$/h is a prerequisite to provide the patch area of lower than 40 cm$^2$ for patient's convenience. Because the higher the rate of an antivomition agent is, the less the patch area required to deliver an effective amount of an antivomiting agent is.

However, the actual skin penetration rate of an antivomiting agent is very low due to the resistance of the lipophilic outmost layer(keratotic layer) of the skin toward drug penetration.

Accordingly, the skin penetration rate of an antivomiting agent is enhanced by way of using a skin penetration enhancer. The skin penetration enhancer reduces the diffusional resistance of the skin and promotes the distribution of the drug into the lipophilic part of the skin by modifying the physicochemical properties of the keratotic layer. N,N-diethyl-m-toluamide increases the solubility of a drug in the keratotic layer and promotes the distribution of the drug into the skin. Particularly, 1 to 5 % by weight, of N,N-diethyl-m-toluamide, when combined with 1 to 5 % by weight of glycerol nionolaurate, synergistically promotes the skin penetration of an antivomiting agent.

The inventive composition may further comprise a terpene. Representative terpenes include /-menthol, menthone, *d*-limonene, 1,S-cineol, nerotidol, carveol and camphor. Among these, /-menthol, *d*-limonene and nerolidol are preferred and /-menthol is more preferred. The terpene, when combined with ethanol, synergistically promotes the skin penetration of an antivomiting agent by enhancing the distribution of the drug into the keratotic layer.

The inventive composition may still further comprise a nonionic surfactant. Representative nonionic surfactants that can be used in the present invention include polyoxyethylene-9-nonylphenyl ether, polyethylene glycol-40 hydrogenated castor oil, polyethylene glycol-35 castor oil and octoxynol-11. Preferred, among these, are polyoxyethylene (n=10) oleyl ether, polyoxyethylene-9-nonylphenyl ether, polyethylene glycol-40 hydrogenated castor oil RH40 and octoxynol-11, and more preferred are polyoxyethylene (n=10) oleyl ether and polyethylene glycol-40 hydrogenated castor oil RH40, polyoxyethylene sorbitan monolauryl ester, polyoxyethylene sorbitan trilauryl ester, polyoxyethylene sorbitan palmityl ester, polyoxyethylene sorbitan stearyl ester, polyoxyethylene sorbitan oleyl ester(Tween [R], ICI), sorbitan monolauryl, sorbitan monopalmityl and sorbitan monostearyl esters(Span[R], ICI). The nonionic surfactant promotes the skin penetration of a drug with a less damage to the skin than an ionic surfactant, i.e., an anionic, cationic or amphoteric surfactant(Water, K.A., *Penetration enhancers and their use in transdermal therapeutic system, Transdermal Drug Delivery,* 212-224, Dekker, (1989); and Eagle et al., /. *Toxicol. cut and Ocular toxicol, 11,* 77-92 (1992)).

The hydrophilic organic solvent which may be used in the present invention is a low molecular weight alcohol such as ethanol, isopropanol, butanol, benzyl alcohol, propylene glycol, glycerin, polyethylene glycol having a molecular weight of 600 or less, diethylene glycol monoethyl ether, triacetin, N-methylpyrrolidone, 2-pyrrolidone, dimethyl sulfoxide, decylmethyl sulfoxide, dioxane, lactone and a mixture thereof. Among these, preferred are a mixture of ethanol and propylene glycol, a mixture of ethanol and glycerin, and a mixture of ethanol and diethylene glycol monoethyl ether and more preferred is a mixture of ethanol and propylene glycol. The hydrophilic organic solvent may be used in an amount ranging from 20 to 80 % by weight, preferably from 20 to 50 % by weight, based on the weight of the matrix. The use of a mixture of 10 to 30 % by weight of ethanol and 10 to 50 % by weight of propylene glycol based on the weight of the matrix is still more preferred.

Ethanol reversibly changes the structure of the keratotic layer by extracting polar lipids therefrom, thereby promoting the skin penetration of an antivomiting agent, and also plays the role of enhancing the solubilization of the antivomiting agent and another watersoluble components of the matrix.

Propylene glycol, when combined with a fatty acid or a terpene, synergically promotes the skin penetration of an antivomiting agent.

The transdermal matrix in the present invention contains 15 to 80 % by weight of water which is distilled water or pH buffer solution.

The composition of the present invention may further comprise a thickener. Representative thickeners include polyvinylpyrrolidone, colloidal silicon dioxide, polyvinyl alcohol, sodium carboxymethylcellulose, hydroxyethylcellulose, hydroxypropylcellulose, hydroxypropylmethylcellulose, carbopol and polyoxyethylene-polyoxypropylene block copolymer(poloxamerR, BASF), and preferred is hydroxypropylcellulose. The thickener may be used in an amount ranging from 1 to 10 % by weight based on the weight of the matrix.

The composition of the present invention may be formulated into a preparation for the transdermal administration, e.g., a patch. Examples of the patch which may be used in the present invention include a reservoir patch and a monolithic matrix patch.

An adhesive matrix patch, another form of patch, may not be suitably employed in the present invention because it can carry only limited amounts of an antivomiting agent and a soluble skin penetration enhancer in its adhesive layer.

The reservoir patch which may be used in the present invention is composed of an impervious protective layer, a reservoir layer containing the composition of the present invention, a drug-permeable membrane, an adhesive layer, a release strip and optionally a peelable disc. The reservoir layer is positioned between the impervious protective layer and one surface of the drug-permeable membrane. The whole surface or an edge portion of one side of adhesive layer is attached to the other surface of the drug-permeable membrane; and optionally the central portion thereof is attached to the peelable disc. The other side of the adhesive layer is attached to the release strip. The peelable disc and the release strip are removed before use.

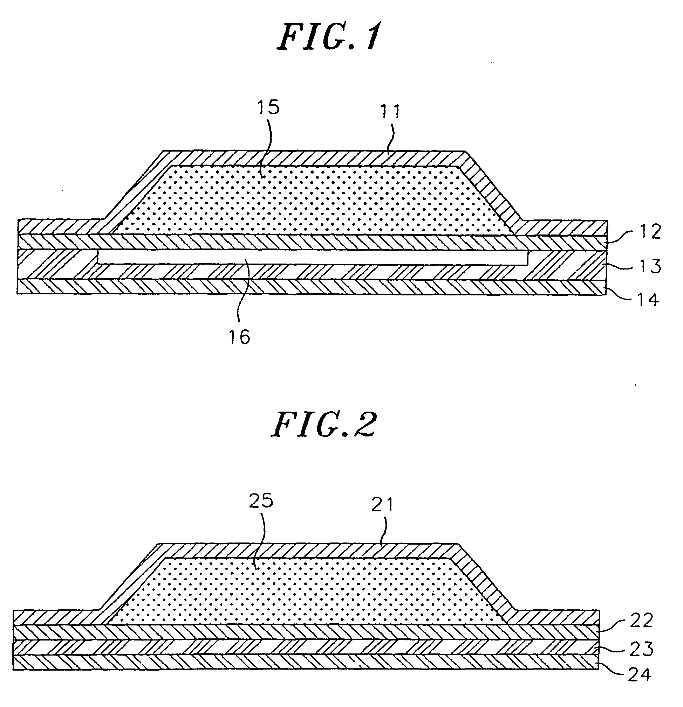

Fig.1 shows an embodiment of the reservoir patch of the present invention, which comprises a reservoir layer(15) containing the inventive composition arranged between an impervious protective layer(11) and a drug-permeable membrane(12), an adhesive layer(13) attached to the bottom surface of the drug-permeable membrane (12) at the edge and to a peelable disc(16) at its central portion, and a release strip(14) attached to the other side of the adhesive layer.

Fig. 2 shows another embodiment of the reservoir patch of the present invention, which comprises a reservoir layer(25) containing the inventive composition positioned between an impervious protective layer(21) and a drug-permeable membrane(22), an adhesive layer(23) attached to the bottom surface of the drug-permeable membrane and a release strip(24) attached to the other side of the adhesive layer.

The impervious protective layer provides the seal to protect the patch form losing volatile component of the composition and it may be made of a polyester, polyethylene or polypropylene. The drug-permeable membrane provides a physical backing of the reservoir layer, which may be porous or nonporous. The drug-permeable membrane may be made of polyethylene or polypropylene and the nonporous membrane may be a film made of ethylenevinyl acetate, silicon rubber or polyolefin. The drug-permeable membrane may be selected to control the release rate of the drug in the composition.

The protective layer and the drug-permeable membrane are sealed by heating the edge portion, thereby enclosing the reservoir layer. The adhesive layer may be made of polyisobutylene, acrylate or silicon.

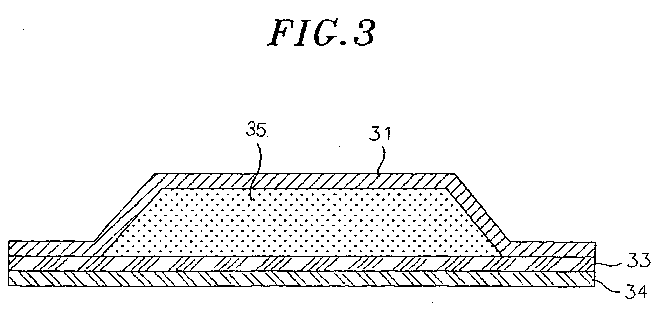

Fig.3 shows an embodiment of the monolithic matrix patch of the present invention, which comprises an impervious protective layer(31), a reservoir layer(35), an adhesive layer(33) and a release strip(34). The reservoir layer(35) is positioned between the impervious protective layer(31) and the adhesive layer(33) which is attached to the release strip(34). The reservoir layer(35) contains the inventive composition dispersed in a polymer, e.g., polyvinylpyrrolidone, polyvinyl alcohol and hydroxyethylcellulose. The reservoir layer may be a hydrogel that comprises 30 to 60 % by weight of water, 25 to 50 % by weight of a composition for the transdermal administration, and 5 to 20 % by weight of a polymer, based on the weight of the reservoir layer.

The patch of the present invention may be prepared by dispersing the inventive composition for the transdermal administration in a polymer solution.

The composition and preparation of the present invention release an antivomiting agent at a rate sufficient for maintaining an effective level of the drug in the blood over a period of 24 to 72 hours at a skin contact area of 10 to 40 cm$^2$.

The following Examples are intended to further illustrate the present invention without limiting its scope.

Further, percentages given below for solid in solid mixture, liquid in liquid, and solid in liquid are on a wt/wt, vol/vol and wt/vol basis, respectively, and all the reactions were carried out at room temperature, unless specifically indicated otherwise.

Reference Example: Determination of Skin Penetration Rate

The skin penetration rate of a drug was determined using a Franz diffusion cell(Model FDC-400, Crown Glass Co., USA) as follows:

A piece of cadaver skin was placed between the donor and receptor compartments of the Franz diffusion cell such that the keratotic layer of the skin faced the donor compartment. The effective area of the skin exposed to the receptor solution was 0.636 cm$^2$. The receptor compartment was filled with 5 ml of distilled water(receptor solution), and stirred at 600 rpm using a magnetic bar while maintaining the temperature at $32\pm0.5°C$ using a thermostat with a circulatory pump. The donor compartment was filled with a degassed transdermal composition and then sealed. The amount of the drug in the receptor compartment was adjusted such that the drug concentration in the receptor solution does not exceed 10 % of the maximum solubility of the drug during the course of the experiment. Samples of the receptor solution was taken at 2, 4, 5, 12, 20 and 24 hours.

The skin penetration rate was determined by the amount of the drug penetrated per unit area of the skin per unit time, according to formula (VI):

$$Js = \frac{1}{A}\left(\frac{dQ}{dt}\right)ss \qquad (VI)$$

wherein Js and A have the same meanings as defined previously; and (dQ/dt)ss is the amount of the drug penetrated per unit time at a steady state.

**Example 1: Preparation of Transdermal Composition (not according to the invention)**

30 % by weight of ethanol, 27 % by weight of propylene glycol, 3 % by weight of oleic acid, and 40 % by weight of water were mixed and then 3 % by weight of ondansetron was added thereto to obtain a transdermal composition(Composition 1).

The procedure of Reference Example was used to determine that the skin penetration rate of ondansetron was 71.4 $\mu$g/cm$^2$/h.

**Comparative Example 1: Preparation of Comparative Composition**

3 % by weight of ondansetron was dissolved in 100 % by weight of water to obtain a comparative composition(Comparative Composition 1). This composition showed no significant penetration of ondansetron through the skin.

**Comparative Example 2: Preparation of Comparative Composition**

3 % by weight of ondansetron was added to a mixture of 20 % by weight of ethanol and 80 % by weight of water to obtain a comparative composition(Comparative Composition 2). The skin penetration rate of ondansetron was 0.7 $\mu$g/cm$^2$/h.

**Comparative Example 3: Preparation of Comparative Composition**

3 % by weight of ondansetron was added to a mixture of 20 % by weight of ethanol, 20 % by weight of propylene glycol and 60 % by weight of water to obtain a comparative composition(Comparative Composition 3). The skin penetration rate of ondansetron was 1.0 $\mu$g/cm$^2$/h.

**Comparative Example 4: Preparation of Comparative Composition**

3 % by weight of ondansetron was added to a mixture of 40 % by weight of ethanol and 60 % by weight of propylene glycol to obtain a comparative composition (Comparative Composition 4). The skin penetration rate of ondansetron was 1.7 $\mu$g/cm$^2$/h.

**Comparative Example 5: Preparation of Comparative Composition**

3 % by weight of ondansetron was added to a mixture of 20 % by weight of ethanol, 5 % by weight of N-methylpyrrolidone and 75 % by weight of water to obtain a comparative composition (Comparative Composition 5). The skin penetration rate of ondansetron was 3.1 $\mu$g/cm$^2$/h.

**Comparative Example 6: Preparation of Comparative Composition**

3 % by weight of ondansetron was added to a mixture of 40 % by weight of ethanol and 60 % by weight of diethylene glycol monoethyl ether to obtain a comparative composition (Comparative Composition 6). The skin penetration rate of ondansetron was 1.4 $\mu$g/cm$^2$/h.

As can be seen from the results of Example 1 and Comparative Examples 1 to 6, Composition 1 with the skin penetration enhancer exhibits a higher skin penetration rate of ondansetron than Comparative Compositions 1 to 7 without the skin penetration enhancer.

**Example 2: Preparation of Transdermal Composition (not according to the invention)**

Added to an aqueous solution containing 20 % by weight of ethanol, 15 % by weight of propylene glycol, 3 % by weight of glycerol monoleate, 2 % by weight of polyoxyethylene(n=10) oleayl ether, and 60 % by weight of water was tropisetron to a concentration of 5 % by weight to obtain a transdermal composition (Composition 2). The skin penetration rate of tropisetron was 30.8 $\mu$g/cm$^2$/h.

**Examples 3 to 42: Preparation of Transdermal Composition**

Transdermal compositions (Compositions 3 to 42) were obtained by repeating the procedure of Example 1 or 2 using the ingredients shown in Table 1 and the skin penetration rate of the active ingredient in each of the compositions was determined by the procedure of Reference Example.

Compositions 3-24, 30, 34-36 and 41-42 of Table 1 are not according to the invention.

## Table 1

| Composition No. | | 3 | 4 | 5 | 6 | 7 | 8 | 9 | 10 | 11 | 12 |
|---|---|---|---|---|---|---|---|---|---|---|---|
| Antivomiting agent* | Tropisetron | | | | | | | | | | |
| | Ondansetron | 3 | 3 | 3 | 3 | 3 | 3 | 3 | 3 | 3 | 3 |
| Ingredient (parts by weight) | Ethanol | 20 | 20 | 20 | 20 | 20 | 20 | 20 | 20 | 20 | 20 |
| | Propylene Glycol | 50 | 50 | 50 | 30 | 38 | 38 | 15 | | 15 · | 29 |
| | Glycerin | | | | | | | | | | |
| | Diethylene Glycol Monoethyl Ether | | | | | | | | | | |
| | Polyethylene Glycol-400 | | | | | | | | 15 | | |
| | Lauric Acid | | | | | | | | 1 | | |
| | Oleic Acid | 3 | 5 | 10 | 1 | 3 | 3 | | | | |
| | Octanol | | | | | | | | | | |
| | n-Nonanol | | | | | 4 | | | | | |
| | Glycerol Monolaurate | | | | | | | | | | |
| | Propylene Glycol Monolaurate | | | | | | | 5 | 5 | 5 | 15 |
| | N,N-Diethyl-m-toluamide | | | | | | | | | | |
| | l-Methol | | | | | | | | | 1 | 1 |
| | d-Limonene | | | | | | | | | | |
| | l-Lactic Acid | | | | | | | | | | |
| | Polyoxyethylene(n=10) Oleyl Ether | | | | | | | | | | |
| | Polyethylene Glycol-40 Hydrogenated Castor Oil | | | | 5 | | | 10 · | 10 | 10 | |
| | Octoxynol-11 | | | | | 5 | 5 | | | | 5 |
| | Water | 27 | 25 | 20 | 44 | 30 | 34 | 50 | 49 | 49 | 30 |
| Skin Penetration Rate (µg/cm²/h) | | 30.7 | 32.2 | 31.8 | 30.4 | 85.8 | 75.7 | 26.6 | 35.3 | 30.8 | 47.1 |

EP 1 150 675 B1

## Table 1 (Continued)

| Composition No. | | 13 | 14 | 15 | 16 | 17 | 18 | 19 | 20 | 21 | 22 |
|---|---|---|---|---|---|---|---|---|---|---|---|
| Antivomiting agent* | Tropisetron | | | | | | | | | | |
| | Ondansetron | 3 | 3 | 3 | 3 | 3 | 6 | 6 | 6 | 6 | 6 |
| Ingredient (parts by weight) | Ethanol | 20 | 20 | 20 | 20 | 20 | 20 | 20 | 20 | 20 | 20 |
| | Propylene Glycol | 25 | | 10 | 25 | 40 | 15 | 15 | 15 | 15 | 15 |
| | Glycerin | 13 | | | | | | | | | |
| | Diethylene Glycol Monoethyl Ether | | | | | | | | | | |
| | Polyethylene Glycol-400 | | | | | | | | | | |
| | Lauric Acid | | | | | | | | | | |
| | Oleic Acid | 3 | | | | | | | | | |
| | Octanol | 1 | | | | | | | | | |
| | n-Nonanol | | 3 | | | | | | | | |
| | Glycerol Monolaurate | | | 3 | 3 | 3 | 1 | 3 | 5 | 3 | 3 |
| | Propylene Glycol Monolaurate | | | | | | | | | 0.5 | |
| | N,N-Diethyl-m-toluamide | | | | | | | | | | |
| | l-Methol | | | | | | | | | | |
| | d-Limonene | | | | | | | | | | 0.5 |
| | l-Lactic Acid | | | | | | | | | | |
| | Polyoxyethylene(n=10) Oleyl Ether | | 2 | 2 | 2 | 2 | 3 | 3 | 3 | 2 | 2 |
| | Polyethylene Glycol-40 Hydrogenated Castor Oil | 5 | | | | | | | | | |
| | Octoxynol-11 | | | | | | | | | | 5 |
| | Water | 33 | 75 | 65 | 50 | 35 | 61 | 59 | 57 | 59.5 | 59.5 |
| Skin Penetration Rate (μg/cm²/h) | | 36.6 | 48.3 | 35.1 | 25.7 | 26.9 | 37.4 | 43.6 | 50.2 | 62.6 | 87.1 |

Table 1 (Continued)

| Composition No. | | 23 | 24 | 25 | 26 | 27 | 28 | 29 | 30 | 31 | 32 |
|---|---|---|---|---|---|---|---|---|---|---|---|
| Antivomiting agent* | Tropisetron | | | | | | | | | | |
| | Ondansetron | 6 | 6 | 6 | 6 | 6 | 6 | 6 | 6 | 6 | 6 |
| Ingredient (parts by weight) | Ethanol | 20 | 20 | 20 | 20 | 20 | 20 | 20 | 20 | 20 | 20 |
| | Propylene Glycol | 15 | 15 | 15 | 15 | 15 | 15 | 15 | 15 | 15 | 15 |
| | Glycerin | | | | | | | | | | |
| | Diethylene Glycol Monoethyl Ether | | | | | | | | | | |
| | Polyethylene Glycol-400 | | | | | | | | | | |
| | Lauric Acid | | | | | | | | | | |
| | Oleic Acid | 0.5 | | | | | | | | | |
| | Octanol | | | | | | | | | | |
| | n-Nonanol | | | | | | | | | | |
| | Glycerol Monolaurate | 3 | 3 | 1 | 1 | 1 | 1 | 2 | | 1 | 1 |
| | Propylene Glycol Monolaurate | | 0.5 | | | | | | | | |
| | N,N-Diethyl-m-toluamide | | | 1 | 3 | 5 | 10 | 3 | 3 | 3 | 3 |
| | l-Methol | | | | | | | | | | |
| | d-Limonene | | 1 | | | | | | | | |
| | l-Lactic Acid | | | | | | | | | 0.3 | 0.5 |
| | Polyoxyethylene(n=10) Oleyl Ether | 2 | 2 | | | | | | | | |
| | Polyethylene Glycol-40 Hydrogenated Castor Oil | | | | | | | | | | |
| | Octoxynol-11 | | | | | | | | | | |
| | Water | 59.5 | 58.5 | 63 | 61 | 59 | 54 | 60 | 62 | 60.7 | 60.5 |
| Skin Penetration Rate (µg/cm²/h) | | 47.1 | 95.8 | 79.2 | 81.4 | 115.3 | 33.2 | 144.1 | 23.2 | 102.6 | 208.8 |

Table 1 (Continued)

| Composition No. | | 33 | 34 | 35 | 36 | 37 | 38 | 39 | 40 | 41 | 42 |
|---|---|---|---|---|---|---|---|---|---|---|---|
| Antivomiting agent* | Tropisetron | | 5 | 5 | 5 | 5 | 5 | 6 | 6 | 6 | 6 |
| | Ondansetron | 6 | | | | | | | | | |
| Ingredient (parts by weight) | Ethanol | 20 | 20 | 20 | 20 | 20 | 20 | 20 | 20 | 20 | 20 |
| | Propylene Glycol | 15 | 15 | 15 | 15 | 15 | 15 | | 15 | | 15 |
| | Glycerin | | | | | | | 15 | 5 | | |
| | Diethylene Glycol MonoethylEther | | | | | | | | | 40 | |
| | Polyethylene Glycol-400 | | | | | | | | | | |
| | Lauric Acid | | | | | | | | | | |
| | Oleic Acid | | | | | | | | | | |
| | Octanol | | | | 1 | | | | | | |
| | n-Nonanol | | | | | | | | | | |
| | Glycerol Monolaurate | 1 | 3 | | 3 | 1 | 1 | 1 | 1 | | 3 |
| | Propylene Glycol Monolaurate | | | 3 | | | | | | 20 | |
| | N,N-Diethyl-m-toluamide | 3 | | | | 1 | 2 | 2 | 2 | | |
| | l-Methol | | | | | | | | | | |
| | d-Limonene | 1 | | | | | | | | | 1 |
| | l-Lactic Acid | | | | | | | | | | |
| | Polyoxyethylene(n=10) Oleyl Ether | | 2 | 4 | 5 | | | | | | 2 |
| | Polyethylene Glycol-40 Hydrogenated Castor Oil | | | | | | | | | | |
| | Octoxynol-11 | | | | | | | | | | |
| | Water | 60 | 60 | 58 | 56 | 63 | 62 | 62 | 57 | 10 | 59 |
| Skin Penetration Rate (µg/cm²/h) | | 55.4 | 30.8 | 39.4 | 42.7 | 69.1 | 85.4 | 64.8 | 67.9 | 49.1 | 33.7 |

Antivomiting agent* : parts by weight based on the weight of the matrix.

Formulation Example 1: Reservoir Patch

A polyester film (Scotchpak 1022, 3M, USA) was coated with an acrylate adhesive (Durotak 87-2196, National Starch) in a thickness of 500 $\mu$m, and the resulting film was dried at room temperature for 30 min. and further dried at 100°C for 15 min. A peelable disc (RayopeelTM, LR4/25, ucb Transpac N.V.) was laminated thereon to obtain an adhesive film.

20 % by weight of ethanol, 15 % by weight of propylene glycol, 1 % by weight of glycerol monooleate, 2 % by weight of N,N-diethyl-m-toluamide and 62 % by weight of water were mixed to obtain a matrix solution. 4.5 % by weight of hydroxypropylcellulose was added to the matrix solution and 6 % by weight of tropisctron was added thereto to obtain a gel.

The gel was placed on a protective film(Scotchpak 1012, polyester film, 3M) in an amount of 150 mg/cm$^2$ and then a drug-permeable membrane (Cotran, microporous polyethylene film, 3M) was placed thereon. The edge portion of the reservoir layer was sealed by heating. The peripheral portion of the drug-permeable membrane was then fixed on the adhesive film and cut in a desired shape to obtain a reservoir patch.

The skin penetration rate of tropisetron from this reservoir patch was 47.2 $\mu$g/cm$^2$/h.

Formulation Example 2: Monolithic Matrix Patch (not according to the invention)

20 % by weight of ethanol, 15 % by weight of polyproylene glycol, 2 % by weight of oleic acid, 3 % by weight of octanol, 5 % by weight of octoxynol and 55 % by weight of water were mixed, and added thereto were 8 % by weight of polyvinylpyrrolidone and 4 % by weight of polyvinyl alcohol dissolved in an distilled water. The resulting solution was stirred vigorously to make it homogeneous. 4 % by weight of hydroxylethylcellulose and 6 % by weight of tropisetron were added thereto and the resultant was homogenized.

The solution thus obtained was placed in a mould to a depth of 2 mm and kept at 4 °C for a day to obtain a hydrogel.

The hydrogel was laminated on an impervious protective layer precoated with an acrylate adhesive(Durotak 87-2196, National Starch) and then a release strip coated with silicon was attached thereon to obtain a monolithic matrix patch.

The skin penetration rate of tropisetron from the monolithic matrix was 21.3 $\mu$g/cm$^2$/h.

**Test Example**

The reservoir patch obtained in Formulation Example 1 was attached to the skin of each of 10 adult subjects for the duration of 24 hours and then dermal irritations such as erythema, edema, itchness and pain were examined and evaluated the degree of irritation by the scale of 0(no irritation) to 4(strong irritation). The test result showed only minor degrees of skin irritation: erythema, 0.3; edema, 0.2; itchness, 0; and pain 0.1.

While the invention has been described with respect to the above specific embodiments, it should be recognized that various modifications and changes may be made to the invention by those skilled in the art which also fall within the scope of the invention as defined by the appended claims.

**Claims**

1.    A transdermal composition comprising (a) a matrix containing (i) 20 to 80 % by weight of a hydrophilic organic solvent, (ii) a mixture of 1 to 5% by weight of N,N-diethyl-m-toluamide and 1 to 5% by weight of glycerol monolaurate as a skin penetration enhancer, and (iii) 15 to 80% by weight of water; and (b) 1 to 15 % by weight, based on the weight of the matrix, of an antivomiting agent selected from the group consisting of tropisetron, ondansetron, granisetron and pharmaceutically acceptable salts thereof.

2.    The transdermal composition of claim 1, wherein the hydrophilic organic solvent is selected from the group consisting of ethanol, isopropanol, butanol, benzyl alcohol, propylene glycol, glycerin, polyethylene glycol having a molecular weight of 600 or less, diethylene glycol monoethyl ether, triacetin, methylpyrrolidone, 2-pyrrolidone, dimethyl sulfoxide, decylmethyl sulfoxide, dioxane, lactone and a mixture thereof.

3.    The transdermal composition of claim 2, wherein the hydrophilic organic solvent is a mixture of ethanol and propylene glycol.

4. The transdermal composition of claim 3, wherein the mixture consists of 10 to 30 % by weight of ethanol and 10 to 50 % by weight of propylene glycol based on the weight of the matrix.

5. The transdermal composition of claim 1, wherein water is distilled water or pH buffer solution.

6. The transdermal composition of claim 1, further comprising a thickener.

7. The transdermal composition of claim 6, wherein the thickener is selected from the group consisting of polyvinylpyrrolidone, colloidal silicon dioxide, polyvinyl alcohol, sodium carboxymethylcellulose, hydroxyethylcellulose, hydroxypropylcellulose, hydroxypropylmethylcellulose, carbopol and polyoxyethylene polyoxypropylene block copolymer.

8. A preparation comprising the transdermal composition according to any one of claims 1 to 7.

**Patentansprüche**

1. Transdermale Zusammensetzung, umfassend

   (a) eine Matrix in (i) 20-80 Gew.-% eines hydrophilen organischen Lösungsmittels, (ii) eine Mischung aus 1-5 Gew.-% N,N-Diethyl-m-toluamid und 1-5 Gew.-% Glycerinmonolaureat als Hautpenetrationsverstärker, und (iii) 15-80 Gew.-% Wasser; und
   (b) 1-15 Gew.-%, bezogen auf das Gewicht der Matrix, eines Antivomitivums, ausgewählt aus der Gruppe, bestehend aus Tropisetron, Ondansetron, Granisetron und ihren pharmazeutisch verträglichen Salzen.

2. Transdermale Zusammensetzung nach Anspruch 1, wobei das hydrophile organische Lösungsmittel ausgewählt wird aus der Gruppe, bestehend aus Ethanol, Isopropanol, Butanol, Benzylalkohol, Propylenglykol, Glycerin, Polyethylenglykol mit einem Mol.-Gew. von 600 oder darunter, Diethylenglykolmonoethylether, Triacetin, Methylpyrrolidon, 2-Pyrrolidon, Dimethylsulfoxid, Decylmethylsulfoxid, Dioxan, Lacton und ihren Mischungen.

3. Transdermale Zusammensetzung nach Anspruch 2, wobei das hydrophile organische Lösungsmittel eine Mischung aus Ethanol und Propylenglykol ist.

4. Transdermale Zusammensetzung nach Anspruch 3, wobei die Mischung aus dem 10-30 Gew.-% Ethanol und 10-50 Gew.-% Propylenglykol, bezogen auf das Gewicht der Matrix, besteht.

5. Transdermale Zusammensetzung nach Anspruch 1, wobei das Wasser destilliertes Wasser oder eine pH-gepufferte Lösung ist.

6. Transdermale Zusammensetzung nach Anspruch 1, die weiterhin ein Verdickungsmittel enthält.

7. Transdermale Zusammensetzung nach Anspruch 6, wobei das Verdickungsmittel ausgewählt ist aus der Gruppe, bestehend aus Polyvinylpyrrolidon, colloidalem Siliciumdioxid, Polyvinylalkohol, Natriumcarboxymethylcellulose, Hydroxyethylcellulose, Hydroxypropylcellulose, Hydroxypropylmethylcellulose, Carbopol und Polyoxyethylen-Polyoxypropylen-Block-Copolymer.

8. Zusammensetzung, enthaltend die transdermale Zusammensetzung nach irgendeinem der Ansprüche 1-7.

**Revendications**

1. Composition transdermique comprenant (a) une matrice contenant (i) entre 20 % et 80 % en poids d'un solvant organique hydrophile, (ii) un mélange de 1 % à 5 % en poids de N,N-diéthyl-m-toluamide et de 1 % à 5 % en poids de monolauréate de glycérine en tant qu'amplificateur de pénétration de la peau, et (iii) entre 15 % et 80 % en poids d'eau ; et (b) entre 1 % et 15 % en poids, par rapport à la masse de la matrice, d'un agent anti vomissement choisi dans le groupe constitué de tropisétron, d'ondasétron, de granisétron et leurs sels pharmaceutiquement acceptables.

2. Composition transdermique selon la revendication 1, dans laquelle le solvant organique hydrophile est choisi dans le groupe constitué d'éthanol, d'isopropanol, de butanol, d'alcool benzylique, de propylèneglycol, de glycérine, de polyéthylèneglycol ayant une masse moléculaire égale ou inférieure à 600, d'éther monoéthylique du diéthylène glycol, de triacétine, de méthylpyrrolidone, de 2-pyrrolidone, de diméthylsulfoxyde, de décylméthylsulfoxyde, de dioxanne, de lactone et un mélange de ceux-ci.

3. Composition transdermique selon la revendication 2, dans laquelle le solvant organique hydrophile est un mélange d'éthanol et de propylèneglycol.

4. Composition transdermique selon la revendication 3, dans laquelle le mélange est constitué de 10 % à 30 % en poids d'éthanol et de 10 % à 50 % en poids de propylèneglycol par rapport à la masse de la matrice.

5. Composition transdermique selon la revendication 1, dans laquelle l'eau est de l'eau distillée ou une solution tampon de pH.

6. Composition transdermique selon la revendication 1, comprenant en outre un épaississant.

7. Composition transdermique selon la revendication 6, dans laquelle l'épaississant est choisi dans le groupe constitué de polyvinylpyrrolidone, de dioxyde de silicium colloïdal, d'alcool polyvinylique, de carboxyméthylcellulose de sodium, d'hydroxyméthylcellulose, d'hydroxypropylcellulose, d'hydroxypropylméthylcellulose, de carbopol et le copolymère séquencé de polyoxyéthylène polyoxypropylène.

8. Préparation comprenant la composition transdermique selon l'une quelconque des revendications 1 à 7.

# FIG.1

# FIG.2

# FIG.3